# DEMANDE DE BREVET EUROPEEN

(11) **EP 1 333 412 A1**
(43) Date de publication de la demande: **06.08.2003**
(21) Numéro de dépôt: 03290168.8
(22) Date de dépôt: 23.01.2003
(51) Int. Cl.: G09B 23/28, A61B 8/08, G10K 11/35

(54) **Simulateur de sonde échographique**

(30) Priorité: 29.01.2002 FR 0201027
(71) Demandeur: Université d'Orleans, 45067 Orleans Cedex 2 (FR)
(72) Inventeur: Poisson, Gérard, 18220 Brecy (FR); Vieyres, Pierre, 18390 St. Germain du Puy (FR); Courreges, Fabien, 37220 L'Ile Bouchard (FR); Smith-Guerin, Nathalie, 18110 Pigny (FR); Novales, Cyril, 18110 Vignoux sous les Aix (FR)
(74) Mandataire: Cabinet Hirsch

(57) **Abrégé**

L'invention concerne un simulateur de sonde échographique comprenant :
- un boîtier (12),
- un piston (14) mobile dans le boîtier (12),
- un organe de rappel (16) du piston (14),
- un capteur de sollicitation (20) du piston (14),
- un localisateur (18) du boîtier (12).

Le simulateur présente l'avantage de servir à l'entraînement de médecins échographistes.

## Description

La présente invention concerne un simulateur de sonde échographique.

Dans le domaine de l'imagerie médicale, l'échographie est connue pour son efficacité, en particulier pour la détection de pathologies. Etant donné que l'efficacité de l'échographie dépend de l'habilité du médecin à réaliser des prises de vue, la réalisation d'une échographie requiert une formation spécifique et longue.

Le document US-A-4 930 512 divulgue un appareil pour solliciter uniformément un transducteur contre un oeil humain. Le transducteur est prévu à une extrémité d'un boîtier interne et est employé pour transmettre et recevoir des ultrasons. Toutefois, l'appareil étant appliqué contre l'oeil d'un patient, un médecin malhabile manipulant l'appareil risque de blesser le patient. Ce document ne divulgue en rien la possibilité de former le médecin.

Le document FR-A-2 796 263 décrit un système télécommandable de positionnement sur un patient d'un dispositif mobile d'observation et/ou d'intervention. Le système permet à un expert distant d'indiquer, en déplaçant une sonde virtuelle le sens des déplacements souhaités d'une sonde réelle. Toutefois, ce document ne décrit aucune caractéristique de la sonde virtuelle ni aucun moyen permettant de fournir au médecin une formation spécifique sur la manipulation de sonde.

Le document US-A-5 609 485 décrit un appareil pour reproduire un examen médical. Ce document prévoit l'utilisation d'une sondé de simulation à ultrasons. Toutefois, ce document ne décrit aucune caractéristique de la sonde virtuelle ni aucun moyen permettant de fournir à l'utilisateur les sensations qu'il percevrait s'il manipulait une sonde réelle d'échographie.

Se pose alors le problème de l'entraînement des médecins. Il existe donc un besoin en un appareil d'entraînement simple de construction qui puisse servir à la formation des médecins.

Pour cela, l'invention concerne un simulateur de sonde échographique comprenant :
- un boîtier,
- un piston mobile dans le boîtier,
- un organe de rappel du piston,
- un capteur de sollicitation du piston,
- un localisateur du boîtier.

La raideur de l'organe de rappel peut varier; l'organe de rappel est par exemple un actionneur linéaire. L'organe de rappel peut aussi être un ressort. L'organe de rappel peut également comprendre l'actionneur linéaire et le ressort.

Avantageusement, le piston est uniquement mobile en translation dans le boîtier.

Le localisateur peut être porté par le boîtier. Le localisateur peut comporter un émetteur et un récepteur. L'émetteur peut être sur le boîtier, la localisation se faisant par rapport au récepteur fixe dans l'environnement. Alternativement, le récepteur est sur le boîtier, la localisation se faisant par rapport à l'émetteur fixe dans l'environnement.

L'invention concerne aussi un procédé d'échographie sur un patient avec un appareil d'échographie, l'appareil comprenant :
- le simulateur de l'invention tel que décrit précédemment,
- un robot portant une sonde échographique sur un bras mobile,
le procédé comprenant les étapes de :
- déplacement du boîtier par un utilisateur,
- transmission du déplacement du boîtier au robot,
- déplacement de la sonde échographique par le bras, par reproduction du déplacement du boîtier.

Avantageusement, le déplacement du boîtier est manuel.

Le procédé peut comprendre en outre une étape de prise de vue par la sonde échographique.

Le robot peut comprendre un capteur des efforts exercés par la sonde échographique sur le patient et le procédé peut comprendre en outre les étapes de :
- mesure des efforts exercés par la sonde échographique sur le patient par le capteur d'effort,
- information de l'utilisateur des efforts exercés.

L'organe de rappel peut être un actionneur linéaire et l'information de l'utilisateur peut commander le mouvement de l'actionneur.

L'invention concerne également un procédé de simulation d'échographie avec le simulateur de l'invention tel que décrit précédemment, le procédé comprenant les étapes de :
- déplacement du boîtier par l'utilisateur,
- extraction de prises de vue d'une banque de données en fonction des déplacements du boîtier,
- affichage des prises de vue.

L'invention présente l'avantage de fournir un simulateur pouvant servir à l'entraînement de médecins. Le simulateur s'applique en particulier à l'entraînement de médecins pratiquant les échographies. L'utilisateur qui manipule le simulateur retrouve les sensations qu'il percevrait s'il manipulait une sonde réelle d'échographie. Le simulateur assure la formation des médecins échographistes sans l'utilisation d'une sonde réelle, ce qui permet d'effectuer une formation plus rapide et moins coûteuse. Par ailleurs, le simulateur peut aussi être appliqué dans un procédé d'échographie à distance.

D'autres caractéristiques et avantages de l'invention apparaîtront à la lecture de la description détaillée qui suit et des modes de réalisation de l'invention, donnés à titre d'exemple uniquement et en références aux dessins qui montrent :
- figure 1, une vue en coupe du simulateur selon l'invention;
- figure 2, une vue en coupe du simulateur de la figure 1 selon une position d'utilisation ;
- figure 3, une vue en coupe du simulateur selon un autre mode de réalisation ;
- figure 4 montre une représentation schématique d'un appareil d'échographie.

On désigne par la suite par « sonde réelle » une sonde servant à la réalisation d'une échographie et à effectuer des prises de vue par ultrasons.

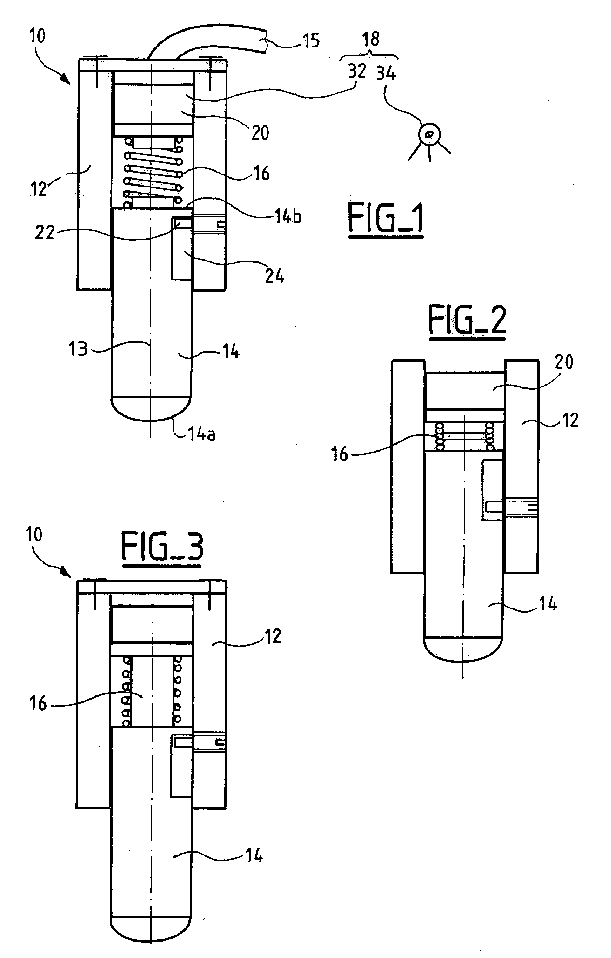

L'invention propose un simulateur de sonde échographique fournissant à l'utilisateur les mêmes sensations que s'il manipulait une sonde réelle et qui est de construction simple. La figure 1 montre une vue en coupe du simulateur selon l'invention. Le simulateur comprend un boîtier 12 dans lequel est mobile un piston 14, et un organe 16 de rappel du piston 14. Le simulateur comprend aussi un capteur de sollicitation 20 du piston 14 et un localisateur 18 du boîtier 12.

Le simulateur de sonde échographique, dans son utilisation, est déplacé sur un objet ; de préférence l'objet a la forme d'un patient, par exemple un mannequin.

Le boîtier 12 a sensiblement la forme et la taille d'une sonde réelle d'échographie. Le boîtier comprend une paroi latérale délimitant une enceinte dans laquelle le piston est mobile. La paroi latérale peut avoir une forme cylindrique ; typiquement, la paroi latérale a une section sensiblement de forme carrée pour une meilleure préhension par l'utilisateur. Le boîtier peut comprendre en outre un fond à une extrémité de la paroi latérale. Le boîtier est alors fermé à une de ses extrémités, l'autre extrémité demeurant ouverte. Le piston 14 est mobile dans le boîtier; avantageusement, le piston est en saillie par rapport au boîtier 12. L'extrémité 14a du piston 14 en saillie du boîtier vient en contact avec l'objet sur lequel l'utilisateur simule l'échographie. De préférence, l'extrémité 14a a une forme sphérique de sorte que le contact entre le piston 14 et l'objet soit en un point et que la rotation du boîtier 12 autour de l'axe longitudinal 13 est facilitée.

Le piston 14 est mobile dans le boîtier 12 selon l'axe longitudinal 13 du boîtier 12. Le piston 14 est mobile en translation dans le boîtier 12. De préférence, le piston 14 est uniquement mobile en translation au moyen d'un ergot 22 dans le boîtier 12 en saillie dans une fente 24 pratiquée dans le piston 14. Suivant l'angle de rotation donné à une sonde échographique réelle autour de son axe longitudinal, on obtient des prises de vue différentes ; l'avantage d'un mouvement uniquement en translation du piston 14 par rapport au boîtier 12 est que l'utilisateur maîtrise l'orientation et la rotation du piston 14. Le piston 14 ne tourne pas de manière incontrôlée autour de l'axe longitudinal 13, mais par rotation du boîtier 12 autour de l'axe longitudinal 13, l'utilisateur donne au piston 14 l'angle de rotation qu'il souhaite. La simulation de l'échographie est d'autant améliorée.

L'organe de rappel 16 du piston 14 permet d'opposer une résistance au déplacement du piston 14 et permet à ce dernier d'occuper une position de repos lorsqu'il n'est pas sollicité en translation. L'organe de rappel 16 permet aussi de donner à l'utilisateur les mêmes sensations que s'il utilisait une sonde réelle sur un patient. L'organe de rappel 16 permet de simuler l'élasticité du corps du patient sur lequel est effectuée l'échographie ; selon que l'échographie est effectuée sur le thorax ou sur l'abdomen du patient, l'utilisateur peut plus ou moins appuyer sur le corps du patient avec la sonde échographique réelle. L'organe de rappel 16 peut être sollicité en compression ou en extension ; principalement, l'organe de rappel 16 est sollicité en compression puisqu'il simule les efforts du patient sur la sonde. La figure 2 montre une vue en coupe du simulateur selon une position d'utilisation. Sur cette figure, l'organe de rappel 16 est en position de compression.

L'organe de rappel 16 est situé entre le fond du boîtier 12 et l'extrémité 14b du piston 14. L'organe de rappel 16 a un comportement élastique ; il s'agit par exemple d'un ressort. Le ressort 16 a un comportement passif. L'avantage d'un ressort est de rendre simple la construction du simulateur. Suivant les patients et suivant les organes où l'échographie est réalisée, l'élasticité du corps varie. Il est alors possible de varier la raideur de l'organe de rappel 16 en remplaçant le ressort par un autre ressort ayant une raideur différente.

La figure 3 montre un autre mode de réalisation du simulateur. Dans ce mode de réalisation, la raideur de l'organe de rappel 16 peut avantageusement varier sans démonter le simulateur 10. L'organe de rappel 16 est par exemple un actionneur linéaire. L'actionneur 16 est relié au piston 14 et provoque le déplacement du piston 14 selon l'axe longitudinal 13. La raideur de l'actionneur 16 peut être commandée sans démonter le simulateur 10. L'utilisation de l'actionneur 16 permet donc de simuler les efforts du patient sur la sonde tels qu'ils se produisent lors d'échographies sur différents patients et/ou en différents organes du corps ; l'actionneur 16 est un organe actif. L'actionneur 16 est par exemple situé entre le fond du boîtier 12 et l'extrémité 14b du piston 14. L'actionneur 16 linéaire est par exemple un moteur linéaire électrique ou un vérin pneumatique. Le vérin peut retenir le mouvement de translation du piston 14 lorsque celui-ci est sollicité en compression vers l'intérieur du boîtier. La résistance à la compression du vérin par le piston 14 est contrôlée par l'utilisateur par action sur le vérin.

Pour améliorer la résistance au déplacement du piston 14, l'organe de rappel peut aussi comprendre l'actionneur linéaire et le ressort. L'actionneur est par exemple dans le ressort pour limiter la place occupée par l'organe de rappel.

Le simulateur ne permet pas de réaliser une prise de vue mais se présente comme une sonde échographique réelle de sorte à donner à l'utilisateur la sensation de manipuler une sonde réelle. Le boîtier 12 et la piston 14 ont ainsi sensiblement le même poids et les mêmes dimensions qu'une sonde réelle. Le boîtier 12 et le piston 14 ont par exemple une longueur de 10 à 13 cm, un diamètre de 3 à 4 cm et un poids de 100 à 200 g.

Le capteur de sollicitation 20 de l'organe de rappel 16 permet à l'utilisateur de contrôler les efforts appliqués à l'objet sur lequel il simule l'échographie. Pour cela, le capteur de sollicitation 20 informe l'utilisateur de la compression (ou de l'extension) par le piston 14 de l'objet. Le capteur de sollicitation 20 donne les six composantes du torseur d'efforts du corps du patient sur le piston 14. Parmi ces paramètres, la composante d'effort suivant l'axe 13 constitue la force normale à la peau du patient et qui est la plus importante à contrôler lors d'un acte d'échographie. L'utilisateur peut ainsi contrôler la pression qu'il exerce sur l'objet et éviter d'exercer des pressions qui provoqueraient une douleur au patient. La simulation de l'échographie en est ainsi encore améliorée. Le capteur de sollicitation 20 est par exemple situé entre le fond du boîtier 12 et l'extrémité de l'organe de rappel 16. Le capteur de sollicitation 20 est alors en contact de l'organe de rappel 16 et le capteur 20 mesure la sollicitation de l'organe de rappel 16 par le piston 14. Dans un autre mode de réalisation, le capteur de sollicitation 20 est à l'extrémité 14a du piston 14 en contact avec l'objet sur lequel s'entraîne l'utilisateur.

Le localisateur 18 permet de détecter les déplacements du boîtier 12. On entend par détection des déplacements du boîtier 12, la détermination de la position du boîtier 12 lorsque le boîtier 12 est immobilisé pour effectuer une prise de vue est simulée, l'itinéraire suivi par le boîtier 12 entre deux positions d'immobilisation ou encore la rotation du boîtier sur lui-même pour simuler une prise de vue selon un autre angle. Par déplacement on entend aussi le fait d'exercer une pression sur l'objet sur lequel on simule l'échographie. Le localisateur 18 permet de mesurer les translations et les rotations du boîtier 12 dans un espace à trois dimensions. Le localisateur 18 relève ainsi 6 paramètres de situation du boîtier 12. Le localisateur 18 peut être porté par le boîtier 12 ou le piston 14 ; il s'agit par exemple d'un accéléromètre. Le localisateur 18 peut aussi être constitué d'un émetteur 32 et d'un récepteur 34. L'émetteur 32 est par exemple sur le boîtier 12 ; l'émetteur 32 peut être porté par le boîtier 12, à l'intérieur ou à l'extérieur de celui-ci. L'émetteur 32 peut aussi être porté par le piston 14. Alternativement, le récepteur 34 est porté par le boîtier 12 ou par le piston 14. Le localisateur 18 est par exemple un capteur magnétique, un capteur à ultrasons ou un capteur optique.

Les déplacements du boîtier 12 et les sollicitations du piston 14 sont recueillis par un calculateur tel qu'un microprocesseur. Pour cela le boîtier 12 est relié au calculateur par exemple par un cordon 15 de connexion flexible tel qu'un cordon réel de liaison pour sonde réelle d'échographie (voir figure 1). L'utilisateur peut être assisté dans les déplacements du boîtier 12, en suivant par exemple les déplacements du boîtier 12 sur un afficheur. Un patient est représenté sur l'afficheur et l'utilisateur déplace le boîtier 12 sur un support tel que le plan d'une table, un tapis de souris d'ordinateur, ou un mannequin.

Le simulateur 10 selon l'invention permet de former les médecins à la pratique des échographies.

Un procédé de simulation va maintenant être expliqué. Des prises de vue effectuées au cours d'une échographie réelle sur un patient sont enregistrées dans une banque de données du calculateur. Le calculateur établit une correspondance entre les déplacements du boîtier 12 par l'utilisateur et les prises de vue enregistrées dans la banque de données ; en fonction des déplacements du boîtier 12 repérés par le localisateur 18, une prise de vue est extraite de la banque de données et est affichée sur un afficheur. L'affichage des prises de vue permet à l'utilisateur d'apprécier la qualité de la simulation de l'échographie effectuée et de d'améliorer son geste. Un tel procédé permet d'entraîner l'utilisateur et de le former en vue d'une pratique future sur des patients dans de réelles conditions.

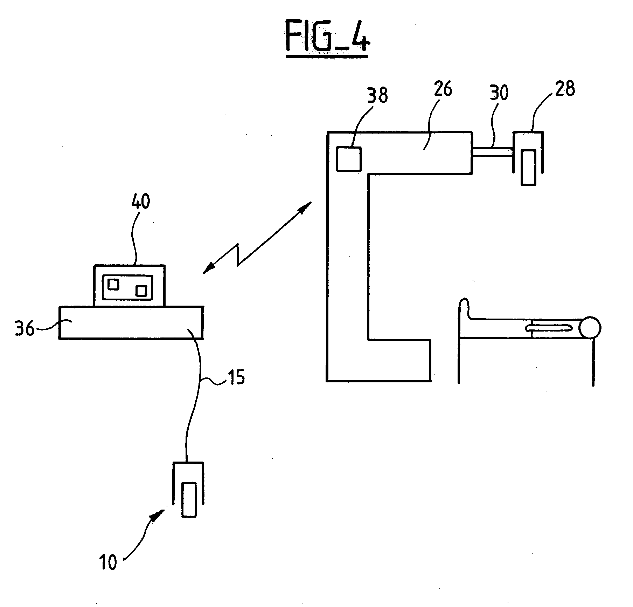

Le simulateur 10 selon l'invention permet aussi de réaliser un procédé d'échographie à distance, ou procédé de télé échographie. On entend par « distance » le fait que l'utilisateur ne déplace pas manuellement la sonde d'échographie réelle sur le patient. Le procédé est mis en oeuvre avec un appareil d'échographie comprenant le simulateur 10 et un robot 26 portant une sonde 28 échographique sur un bras 30 mobile. La figure 4 montre une représentation schématique de l'appareil d'échographie. La sonde 28 échographique réelle est une sonde classique permettant d'obtenir des prises de vue par ultrasons du patient. Le bras 30 est mobile en translation selon trois directions et en rotation autour de ces trois directions. Le simulateur 10 est connecté à un calculateur 36 ; le calculateur 36 est relié au robot 26 qui comporte également un calculateur 38. Le calculateur 36 reçoit les déplacements du boîtier 12 mesurés par le localisateur 18 ainsi que les sollicitations du piston 14 mesurées par le capteur 20 de sollicitation et les transmet au calculateur 38 du robot 26. Le procédé d'échographie comprend une étape de déplacement du boîtier 12 ; l'utilisateur manipule manuellement le boîtier 12 sur un objet tel qu'un mannequin. Le procédé comprend ensuite une étape de transmission du déplacement du boîtier 12 au robot, et en particulier au calculateur 38 du robot 26. Le robot 26 est ainsi informé des paramètres caractéristiques des déplacements du boîtier 12 qu'il reproduit dans une étape suivante de déplacement de la sonde 28 par mouvement du bras 30. L'utilisateur réalise alors des prises de vue du patient à l'aide de la sonde 28. Les images échographiques des organes auscultés sont transmises à l'utilisateur.

Le simulateur 10 et le robot 26 sont reliés entre eux par des moyens connus de communication tels que par le réseau Internet ou tout autre moyen. Les moyens de communication seront choisis de sortes à ce que les informations entre le simulateur 10 et le robot 26 soient transmises en temps réel. L'utilisateur peut ainsi effectuer une échographie à distance avec le simulateur : les prises de vue recueillies par la sonde 28 lui sont transmises par des moyens de communication et affichées sur un afficheur 40, ce qui lui permet de rectifier la prise de vue en déplaçant de manière convenable le boîtier 12 ou en modifiant son incidence.

Selon un mode de réalisation de l'appareil d'échographie, la sonde 28 est munie d'un capteur des efforts exercés par la sonde échographique 28 sur le patient. Le procédé d'échographie comprend alors une étape de mesure des efforts exercés par la sonde 28 sur le patient par le capteur des efforts ; l'utilisateur est ensuite informé des efforts exercés. Il peut en conséquence rectifier le mouvement de la sonde 28 réelle avec le simulateur 10.

Ce mode d'information de l'utilisateur peut être visuel sur l'afficheur 40. Avantageusement, l'information est tactile. L'organe de rappel est l'actionneur linéaire et l'information de l'utilisateur est alors transmise et commande le mouvement de l'actionneur linéaire 16 intégré au simulateur 10 pour constituer une commande dite à « retour d'effort ». Dans cette situation, l'utilisateur ressent dans le simulateur 10 le même effort que celui réellement appliqué sur le patient.

## Revendications

1. Un simulateur de sonde échographique comprenant :
- un boîtier (12),
- un piston (14) mobile dans le boîtier (12),
- un organe de rappel (16) du piston (14),
- un capteur de sollicitation (20) du piston (14),
- un localisateur (18) du boîtier (12).

2. Le simulateur selon la revendication 1, **caractérisé en ce que** la raideur de l'organe de rappel (16) varie.

3. Le simulateur selon la revendication 2, **caractérisé en ce que** l'organe de rappel (16) est un actionneur linéaire.

4. Le simulateur selon la revendication 1, **caractérisé en ce que** l'organe de rappel (16) est un ressort.

5. Le simulateur selon la revendication 2, **caractérisé en ce que** l'organe de rappel (16) comprend un actionneur linéaire et un ressort.

6. Le simulateur selon l'une des revendications 1 à 5, **caractérisé en ce que** le piston (14) est uniquement mobile en translation dans le boîtier (12).

7. Le simulateur selon l'une des revendications 1 à 6, **caractérisé en ce que** le localisateur (18) est porté par le boîtier (12).

8. Le simulateur selon l'une des revendications 1 à 6, **caractérisé en ce que** le localisateur (18) comporte un émetteur (32) et un récepteur (34).

9. Le simulateur selon la revendication 8, **caractérisé en ce que** l'émetteur (32) ou le récepteur (34) est sur le boîtier (12).

10. Procédé d'échographie sur un patient avec un appareil d'échographie, l'appareil comprenant :
- le simulateur (10) selon l'une des revendications 1 à 9,
- un robot (26) portant une sonde (28) échographique sur un bras (30) mobile,
le procédé comprenant les étapes de :
- déplacement du boîtier (12) par un utilisateur,
- transmission du déplacement du boîtier (12) au robot (26),
- déplacement de la sonde (28) échographique par le bras (30), par reproduction du déplacement du boîtier (12).

11. Procédé selon la revendication 10, **caractérisé en ce que** le déplacement du boîtier (12) est manuel.

12. Procédé selon la revendication 10 ou 11, **caractérisé en ce qu'**il comprend en outre une étape de prise de vue par la sonde (28) échographique.

13. Procédé selon l'une des revendications 10 à 12, **caractérisé en ce que** le robot (26) comprend un capteur des efforts exercés par la sonde (28) échographique sur le patient.

14. Procédé selon la revendication 13, **caractérisé en ce qu'**il comprend en outre les étapes de :
- mesure des efforts exercés par la sonde (28) échographique sur le patient par le capteur d'effort,
- information de l'utilisateur des efforts exercés.

15. Procédé selon la revendication 14, **caractérisé en ce que** l'organe de rappel (16) est un actionneur linéaire et que l'information de l'utilisateur commande le mouvement de l'actionneur.

16. Procédé de simulation d'échographie avec un simulateur selon l'une des revendications 1 à 7, le procédé comprenant les étapes de :
- déplacement du boîtier (12) par l'utilisateur,
- extraction de prises de vue d'une banque de données en fonction des déplacements du boîtier (12),
- affichage des prises de vue.
